# EUROPEAN PATENT SPECIFICATION

(11) **EP 2 416 705 B1**
(45) Date of publication and mention of the grant of the patent: **18.01.2017**
(21) Application number: 10713248.2
(22) Date of filing: 15.03.2010
(51) Int. Cl.: A61B 5/08, A61B 5/00, A61M 16/00

(54) **SYSTEM AND METHOD FOR MONITORING PULMONARY CONGESTION**
SYSTEM UND VERFAHREN ZUR ÜBERWACHUNG VON LUNGENSTAUUNG
SYSTÈME ET PROCÉDÉ POUR SURVEILLER LA CONGESTION PULMONAIRE

(30) Priority: 08.04.2009 US 167560 P
(43) Date of publication of application: 15.02.2012
(73) Proprietor: Koninklijke Philips N.V., 5656 AE Eindhoven (NL)
(72) Inventor: PITTMAN, Stephen, Dalton, Briarcliff Manor, New York 10510-8001 (US); WITT, Erik, Kurt, Briarcliff Manor, New York 10510-8001 (US); MECHLENBURG, Douglas, Briarcliff Manor, New York 10510-8001 (US)
(74) Representative: van Velzen, Maaike Mathilde
(86) International application number: PCT/IB2010/051116
(87) International publication number: WO 2010/116276

(56) References cited:
- WO-A1-2005/079897
- WO-A1-2006/068622
- US-A1- 2005 115 561
- US-A1- 2005 142 070
- US-A1- 2006 020 295

## Description

The invention relates to the identification of pulmonary congestion in a subject to facilitate preemptive treatment of heart failure associated with identified pulmonary congestion.

Heart Failure is considered to be prevalent both internationally and in the United States. It is estimated that over 5 million Americans have diagnosed heart failure and many more remain undiagnosed. Estimates suggest the prevalence doubled in a recent 10 year period. According to the Heart Failure Society of America between 400,000 and 700,000 new cases are diagnosed in the US each year. Heart failure is a progressive disorder that is initially managed in most cases by the primary care physician or general cardiologist. However, as the condition progresses to a point where more active medical management is required or heart transplantation is being considered, heart failure specialists usually become involved with patient care.

Left ventricular systolic heart failure occurs when there is failure of left ventricular contractile properties, whereas diastolic heart failure occurs when there is failure of relaxing and filling the left ventricle. Pulmonary edema (congestion) can occur when venous return exceeds cardiac output due to left ventricular dysfunction resulting in an elevated pulmonary capillary hydrostatic pressure and transudation of fluid into the pulmonary interstitium.

The annual financial burden of heart failure on the US Healthcare system exceeds $38B according to a 1994 report. This same report estimates 60% of the cost is related to the 6.5M hospital days associated with hospitalization to manage acute heart failure decompensation. A recent report indicates that the increase in fluid load (pulmonary congestion) that precedes most hospitalizations for acutely decompensated congestive heart failure begins many days (mean 18.3±10.6 days) before the actual hospitalization and almost as many days (mean 15.3±10.6 days) before symptoms first appear.

Documents US2005115561 A1, US2005142070 A1 and US2006020295 A1 disclose prior art systems for monitoring pulmonary congestion in a subject.

The present invention relates to a system configured to monitor pulmonary congestion according to claim 1. Said system distinguishes itself from the prior art by the feature indicated in the characterizing portion.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. In one embodiment of the invention, the structural components illustrated herein are drawn to scale. It is to be expressly understood, however, that the drawings are for the purpose of illustration and description only and are not a limitation of the invention. In addition, it should be appreciated that structural features shown or described in any one embodiment herein can be used in other embodiments as well. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.
FIG. 1 illustrates a system configured to detect pulmonary congestion in a subject, according to one or more embodiments of the invention.
FIG. 2 illustrates plots of lung compliance and % Cheyne-Stokes respiration versus time, in accordance with one or more embodiments of the invention.
FIG. 3 illustrates a graphical user interface generated for a caregiver, according to one or more embodiments of the invention.
FIG. 4 illustrates a system configured to detect pulmonary congestion in a subject, according to one or more embodiments of the invention.
FIG. 5 illustrates a system configured to detect pulmonary congestion in a subject, according to one or more embodiments of the invention.
FIG. 6 illustrates a method of monitoring pulmonary congestion in a subject, in accordance with one or more embodiments of the invention.

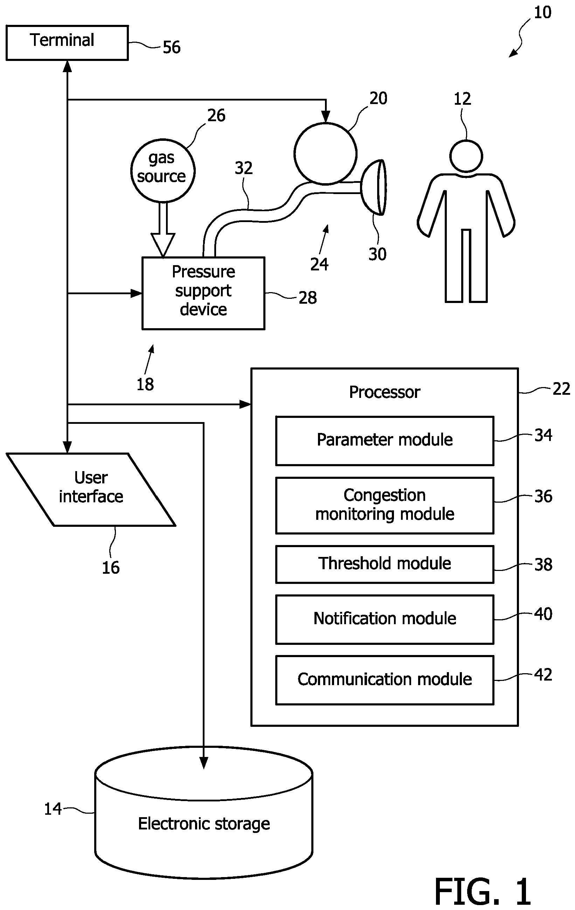

FIG. 1 illustrates a system 10 configured to detect pulmonary congestion in a subject 12. System 10 detects pulmonary congestion in subject 12 based on one or more parameters of the breathing of the subject 12. The detection of pulmonary congestion in subject 12 may be relatively passive for subject 12. This may enhance the convenience and/or comfort of compliance to a detection regime by subject 12. By detecting pulmonary congestion in subject 12, potential episodes of heart failure may be identified and/or averted. System 10 may be configured to facilitate treatment of subject 12 to remediate detected pulmonary congestion and/or avert potential episodes of heart failure. For example, system 10 may provide enhanced access for a caregiver to detections of parameters related to pulmonary congestion in subject 12. In one embodiment, system 10 includes one or more of electronic storage 14, a user interface 16, a pressure generator 18, one or more sensors 20, a processor 22, and/or other components.

In one embodiment, electronic storage 14 comprises electronic storage media that electronically stores information. The electronically storage media of electronic storage 14 may include one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 14 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 14 may store software algorithms, computer program modules, information determined by processor 22, information received via user interface 16, and/or other information that enables system 10 to function properly. Electronic storage 14 may be a separate component within system 10, or electronic storage 14 may be provided integrally with one or more other components of system 10. Although electronic storage 14 is illustrated in FIG. 1 as a single entity, in one embodiment, electronic storage 14 includes a plurality of electronic media divided amongst a plurality of different devices and/or components within system 10.

User interface 16 is configured to provide an interface between system 10 and one or more users. The one or more users may include a subject 12 and/or one or more caregivers (*e.g*., clinicians, doctors, nurses, medical administrators, pharmacists, *etc*.). The interface between system 10 and the one or more users enables the one or more users to provide information to and receive information from system 10. This enables data, results, and/or instructions and any other communicable items, collectively referred to as "information," to be communicated between the one or more users and system 10. Examples of interface devices suitable for inclusion in user interface 16 include a keypad, buttons, switches, a keyboard, knobs, levers, a display screen, a touch screen, speakers, a microphone, an indicator light, an audible alarm, and/or a printer. User interface 16 may include one or more graphical user interfaces provided to users via electronic processing platforms (*e.g.,* a desktop computer, a laptop computer, a handheld computer, a mobile communication device, *etc**.***)*.* The graphical user interface provided to users may be accessible via a web-based information portal.

It is to be understood that other communication techniques, either hardwired or wireless, are also contemplated by the present invention as user interface 16. For example, the present invention contemplates that user interface 16 may be integrated with a removable storage interface provided by electronic storage 14. In this example, information may be loaded into system 10 from removable storage *(e.g.,* a smart card, a flash drive, a removable disk, *etc.)* that enables the user(s) to customize the implementation of system 10. Other exemplary input devices and techniques adapted for use with system 10 as user interface 16 include, but are not limited to, an RS-232 port, RF link, an IR link, modem (telephone, cable or other). In short, any technique for communicating information with system 10 is contemplated by the present invention as user interface 16.

Pressure generator 18 is configured to generate a pressurized flow of breathable gas for delivery to the airway of subject 12 by a circuit 24. One or more parameters of the pressurized flow of breathable gas generated by pressure generator 18 may be controlled by pressure generator 18 for therapeutic purposes, and/or to enable determination of parameters of the breathing of subject 12. For example, pressure generator 18 may control one or more of the pressure, the flow rate, the composition, and/or other parameters of the pressurized flow of breathable gas. In one embodiment, pressure generator 18 includes a gas source 26 and a pressure support device 28.

Gas source 26 includes a body or bodies of gas from which pressure support device 28 generates the pressurized flow of breathable gas that is delivered to subject 12. Gas source 26 may include any supply of breathing gas, such as, for example, ambient atmosphere, a tank of pressurized gas, a wall gas source, and/or other bodies of breathable gas. The breathing gas from gas source 26 can be any breathable gas, such as air, oxygen, an oxygen mixture, a mixture of a breathing gas and a medication, which can be in gaseous form (*e.g.,* nitric oxide, nebulized, *etc*.), and/or other breathable gases.

Pressure support device 28 includes one or more mechanisms for controlling one or more parameters of the flow of breathable gas released from pressure support device 28 to circuit 24. For example, pressure support device 28 may include one or more of a valve, a blower, a piston, a bellows, and/or other mechanisms for controlling one or more parameters of the flow of breathable gas.

In one embodiment, pressure support device 28 controls one or more of the parameters of the pressurized flow of breathable gas in accordance with a predetermined algorithm that provides a therapeutic benefit to subject 12. By way of non-limiting example, pressure support device 28 may control one or more of the pressure and/or flow rate of the breathable gas to facilitate respiration, support the airway of subject 12, to adjust the composition of gas breathed by subject 12, and/or for other therapeutic purposes.

Circuit 24 defines a gas flow path between pressure generator 18 and the airway of subject 12. As such, circuit 24 is configured to deliver the pressurized flow of gas from pressure generator 18 to the airway of subject 12. In one embodiment, circuit 24 includes one or more of an interface appliance 30 and a conduit 32.

Interface appliance 30 is configured to provide gas to and receive gas from the airway of subject 12. Interface appliance 30 may include may include either an invasive or non-invasive appliance for communicating gas between circuit 24 and the airway of subject 12. For example, interface appliance 30 may include a nasal mask, nasal/oral mask, total face mask, nasal cannula, endotracheal tube, LMA, tracheal tube, and/or other interface appliance.

Conduit 32 forms a flow path between pressure support device 18 and interface appliance 30. In one embodiment, conduit 32 is flexible.

Although circuit 24 is illustrated in FIG. 1 as a single-limbed circuit for communicating a pressurized flow of breathable gas with the airway of subject 12, this is not intended to be limiting. In one embodiment circuit 24 is a double-limbed circuit with a separate portion configured to convey gas away from the airway of subject 12.

In one embodiment, some or all of the structure and function attributed to electronic storage 14 and/or user interface 16 may be incorporated into system 10 at pressure support device 18. For example, pressure support device 18 may include a positive pressure support system configured to provide positive airway support to subject 12 during bedtime. The positive pressure support system may include a user interface that enables subject 12 to provide information to and/or receive information from the positive pressure support system. This user interface may provide at least some of the structure and function attributed to user interface 16. The positive pressure support system may include one or more electronic storage media that store, for instance, algorithms, modules, and/or data associated with the therapy provided to subject 12. These same one or more electronic storage media may provide at least some of the structure and function attributed to electronic storage 14.

The sensors 20 are configured to monitor one or more parameters indicative of pulmonary congestion and/or general pulmonary well-being health in subject 12. For example, sensors 20 may include one or more sensors configured to generate output signals conveying information related to one or more parameters of the gas breathed by subject 12. Such sensors may include, for instance, one or more of a pressure sensor, a flowmeter, a capnometer, and/or other sensors configured to generate output signals conveying information related to one or more parameters of the gas breathed by subject 12. The sensors 20 configured to monitor parameters of gas breathed by subject 12 may be disposed in system 10 so as to be in communication with gas within circuit 24. The sensors 20 in communication with gas within circuit 24 include sensors integrally disposed in a positive pressure support system functioning as pressure support device 18.

The incorporation of at least some of sensors 20 into system 10 to communicate with gas within circuit 24 may enhance the passivity of a testing regimen to subject 12. For example, if subject 12 already uses pressure support device 18 for therapeutic purposes (*e.g.,* for airway support during bedtime), a testing regimen based at least in part on parameters of gas within circuit 24 detected by sensors 20 would not require extra effort or steps on the part of subject 12.

The example of sensors 20 including sensors configured to monitor parameters of gas breathed by subject 12 is not intended to be limiting. The sensors 20 may include other types of sensors. For instance, sensors 20 may include one or more of an electrocardiograph, a weight scale, a blood pressure monitor, an impedance sensor, and/or other sensors.

Processor 22 is configured to provide information processing capabilities in system 10. As such, processor 22 may include one or more of a digital processor, an analog processor, a digital circuit designed to process information, an analog circuit designed to process information, a state machine, and/or other mechanisms for electronically processing information. Although processor 22 is shown in FIG. 1 as a single entity, this is for illustrative purposes only. In some implementations, processor 22 includes a plurality of processing units. These processing units may be physically located within the same device, or processor 22 may represent processing functionality of a plurality of devices operating in coordination. The plurality of devices may be operatively linked. The operative link(s) between the plurality of devices may be accomplished via dedicated connection, networked connection, wireless connection, wired connection, and/or via other electronic communication connections. As such, the linked devices may be physically proximate to each other, and/or physically remote from each other. For example, the plurality of devices may include pressure support device 18, an electronic processing platform associated with subject 12, an electronic processing platform associated with a caregiver of subject 12, a server provided by service provider, and/or other devices.

As is shown in FIG. 1, in one embodiment, processor 22 is configured to execute one or more computer program modules to provide the functionality attributed to processor 22 herein. The one or more computer program modules may include one or more of a parameter module 34, a congestion monitor module 36, a threshold module 38, a notification module 40, a communication module 42, and/or other modules. Modules 34, 36, 38, 40, and/or 42 may be implemented in software; hardware; firmware; some combination of software, hardware, and/or firmware; and/or otherwise implemented. It should be appreciated that although modules 34, 36, 38, 40, and/or 42 are illustrated in FIG. 1 as being co-located within a single processing unit, in implementations in which processor 22 includes multiple processing units, modules 34, 36, 38, 40, and/or 42 may be located remotely from the other modules. Further, the description of the functionality provided by the different modules 34, 36, 38, 40, and/or 42 described below is for illustrative purposes, and is not intended to be limiting, as any of modules 34, 36, 38, 40, and/or 42 may provide more or less functionality than is described. For example, one or more of modules 34, 36, 38, 40, and/or 42 may be eliminated, and some or all of its functionality may be provided by other ones of modules 34, 36, 38, 40, and/or 42. As another example, processor 22 may execute one or more additional modules that may perform some or all of the functionality attributed below to one of modules 34, 36, 38, 40, and/or 42.

Parameter module 34 is configured to receive output signals from sensors 20, and to determine one or more parameters that are indicative of pulmonary congestion in subject 12 based on the received output signals. As pulmonary congestion increases, interstitial fluid in and around the lungs tends to increase. This increase in interstitial fluid typically impacts respiration. For example, the increased interstitial fluid decreases lung compliance, and causes difficulty breathing. The increased difficulty in breathing may manifest itself in the form of nocturnal hypoxia and/or sleep disordered breathing. In particular, pulmonary congestion tends to increase Cheyne-Stokes respiration at night. Therefore, the one or more parameters determined by parameter module 34 include at least one parameter of the breathing of subject 12 likely to be impacted by pulmonary congestion. For instance, the at least one parameter of the breathing of subject 12 may include lung compliance, identification of Cheyne-Stokes respiration, forced vital capacity, forced inspiratory volume in 1 second, forced expiratory volume in 1 second, peak inspiratory flow, peak expiratory flow, expired CO₂, minute ventilation, respiratory rate, mean and standard deviation of breath period, mean and standard deviation of tidal volume, and/or other parameters of the breathing of subject 12. In one embodiment, the at least one breathing parameter of subject 12 determined by parameter module 34 includes a rate of change of a breathing parameter (*e.g.,* a rate of change of one of the parameters enumerated above).

In one embodiment, the one or more parameters determined by parameter module 34 include at least one non-breathing parameter. The at least one non-breathing parameter may include, for example, body weight, a vascular parameter (*e.g.,* blood pressure), and/or other non-breathing parameters indicative of pulmonary congestion.

In one embodiment, parameter module 34 controls user interface 16, pressure support device 18, and/or other components of system 10 to facilitate determinations of at least one of the one or more parameters. Determination of various parameters of the breathing of subject 12 (and/or other parameters indicative of pulmonary congestion) may require cooperation of subject 12, specific control over the pressurized flow of breathable gas generated by pressure support device 18, and/or other circumstances that can be manipulated by the components of system 10.

By way of example, determinations of lung compliance, forced vital capacity, forced inspiratory volume in 1 second, and/or other breathing parameters require one or more parameters of the pressurized flow of breathable gas generated by pressure support device 18 to be manipulated in a specific manner. To facilitate determinations of such parameters, parameter module 34 causes pressure support device 18 to override the treatment algorithm generally used to control parameters of the pressurized flow of breathable gas to provide subject 12 with airway support at bedtime. The parameter module 34 may control pressure support device 18 in this manner at predetermined intervals, in response to predetermined events, and/or in accordance with a predetermined testing regimen.

As another example, determinations of forced expiratory volume in 1 second, body weight, blood pressure, and/or other parameters determined by parameter module 34 may require affirmative action on the part of subject 12. The parameter module 34 may control user interface 16 to provide cues to subject 12 that prompt subject 12 to take the appropriate action to enable determination of the appropriate parameter(s). For instance, to facilitate a determination forced expiratory volume in 1 second, parameter module 34 may control user interface 16 to provide subject 12 with a cue that prompts subject 12 to exhale as hard as possible for 1 second. To facilitate a determination of body weight, parameter module 34 may control user interface 16 to provide subject 12 with a cue that prompts subject 12 to mount a body weight scale associated with system 10.

In one embodiment, the parameters determined by parameter module 34 include parameters based on subjective inputs provided by subject 12 to system 10 via user interface 16. For example, the subjective inputs may include responses to quality of life queries related to pulmonary congestion and/or heart failure. Such quality of life queries may, for example, request subject 12 to rate effort in breathing, shortness of breath, fatigue, and/or other subjective perceptions of health.

Parameter module 34 manages the storage of the determined one or more parameters to electronic storage 14. This may include storing the determined one or more parameters with information identifying other information related to the user and/or the parameter determined. For example, a parameter determination may be stored with an identity of subject 12, a date/time stamp indicating the date and/or time that the parameter was determined, and/or other information. Storing the determined one or more parameters may include storing the determined parameter(s) locally on a device connected directly with sensors 20, and/or may include storing the determined parameters remotely. For instance, in one embodiment, parameters determined by parameter module 34 are stored in a central information repository (e.g., on a server) that is located remote from the device(s) connected directly with sensors 20. The central information repository may be accessible to subject 12 and/or a caregiver.

The congestion monitor module 36 is configured to monitor pulmonary congestion in subject 12 based on the one or more parameters determined by parameter module 34. Monitoring pulmonary congestion in subject 12 may include identifying pulmonary congestion in subject 12 based on the one or more parameters determined by parameter module 34.

Although a variety of algorithms may be implemented to identify signs of pulmonary congestion in subject 12, in one embodiment, congestion monitor module 36 compares the one or more parameters determined by parameter module 34 with one or more thresholds to identify pulmonary congestion. If a predetermined number and/or combination of the one or more parameters breaches the corresponding thresholds, congestion monitor module 36 identifies pulmonary congestion in subject 12.

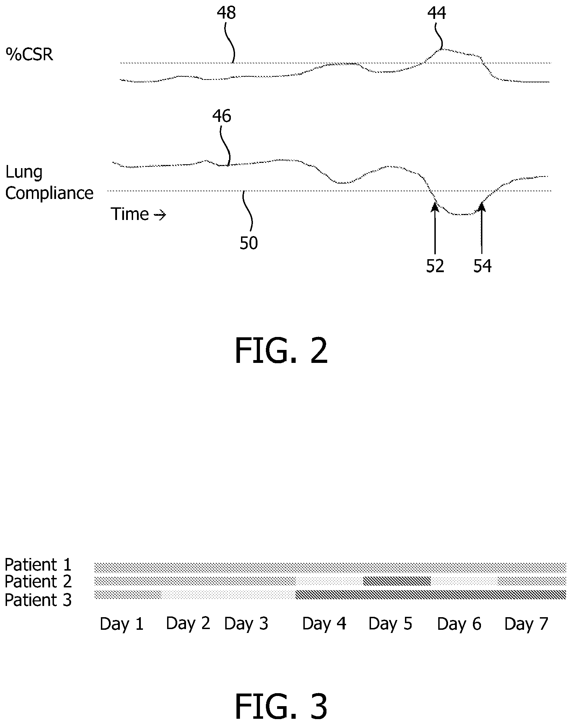

By way of illustration, FIG. 2 plots 44 and 46 of % Cheyne-Stokes respiration and lung compliance, respectively, versus time. FIG. 2 also illustrates thresholds 48 and 50, corresponding to % Cheyne-Stokes respiration and lung compliance, respectively. By comparing the lung compliance of a patient (represented by plot 46) with the corresponding threshold 50, clinically significant pulmonary congestion may be identified. Similarly, by comparing the percentage of time that the subject experiences Cheyne-Stokes respiration (represented by plot 44) with the corresponding threshold 48, clinically significant pulmonary congestion may be identified.

In one embodiment, once a parameter, such as lung compliance, breaches its corresponding threshold parameter, the module monitoring the parameter (*e.g.,* congestion monitor module 36 shown in FIG. 1 and described above) immediately identifies pulmonary congestion in the subject. In one embodiment, the module monitoring the parameter only identifies pulmonary congestion in the subject if the parameter remains across the threshold for a predetermined amount of time. By way of illustration, in FIG. 2, at a first point in time 52, lung compliance 46 crosses threshold 50. At a second point in time 54, pulmonary congestion is identified because lung compliance 46 has remained across threshold 50 for a predetermined amount of time (and/or number of measurements).

In one embodiment, when a parameter breaches its threshold, the module monitoring the parameter identifies a preliminary stage of pulmonary congestion. Based on subsequent determinations of the parameters, the module may identify stages of pulmonary congestion of increasing seriousness, and/or may identify an absence of pulmonary congestion (*e.g.,* if the parameter moves back across the threshold). For example, if the parameter remains across the threshold and/or breaches additional thresholds the module may identify stages of pulmonary congestion of increasing seriousness.

Returning to FIG. 1, in one embodiment, congestion monitor module 36 identifies pulmonary congestion based on analysis of individual parameters. In one embodiment, congestion monitor module 36 blends analysis of a plurality of parameters determined by parameter module 34 in identifying pulmonary congestion. For example, congestion monitor module 36 may not identify pulmonary congestion in subject 12 unless a predetermined number of parameters have crossed their thresholds. As another example, congestion monitor module 36 may identify different stages or degrees of pulmonary congestion based on the number of parameters that have crossed their thresholds.

The description of congestion monitor module 36 analyzing the parameter(s) determined by parameter module 34 with respect to one or more thresholds to identify pulmonary congestion in subject 12 is not intended to be limiting. Other types of analysis may be implemented to identify pulmonary congestion in subject 12 from parameters determined by parameter module 34 that indicate pulmonary congestion, without departing from the scope of this disclosure. The implementation of threshold analysis is provided for illustrative purposes.

The threshold module 38 is configured to manage one or more thresholds. The one or more thresholds may be implemented by congestion monitor module 36 to identify pulmonary congestion in subject 12 (*e.g.,* in the manner described above). Managing the one or more thresholds may include enabling a user (*e.g.,* subject 12, a caregiver, *etc*.) to customize and/or configure one or more of the thresholds. Customization of one or more of the thresholds includes configuring the customized thresholds to enhance the identification of pulmonary congestion, enhance convenience and/or comfort of testing, and/or providing other enhancements specifically tailored to subject 12.

In one embodiment, one or more thresholds managed by threshold module 38 are static (*e.g.,* as illustrated by thresholds 48 and 50 in FIG. 2). In one embodiment, one or more thresholds managed by threshold module 38 are adaptive and dynamic. For instance, a threshold corresponding to lung compliance (or some other parameter) may adapt based on determinations of % Cheyne-Stokes respiration (or some other parameter) made by parameter module 34. By way of example, if the % Cheyne-Stokes respiration reaches a predetermined level (*e.g.,* the threshold corresponding to % Cheyne-Stokes respiration), the threshold corresponding to lung compliance may be adjusted such that a smaller change in lung compliance will cause the compliance to breach its threshold. This adjustment of the threshold corresponding to lung compliance will increase the sensitivity of system 10 with respect to changes in the lung compliance indicative of pulmonary congestion if % Cheyne-Stokes respiration indicates that pulmonary congestion is likely.

In one embodiment, threshold module 38 manages a threshold such that the threshold adapts dynamically to previous determinations of the parameter corresponding to the threshold. For example, if the lung compliance of subject 12 (or some other parameter) undergoes several spikes with it breaches its threshold and then returns over the threshold before pulmonary congestion is identified, threshold module 38 will make adjustments to increase the sensitivity of system 10 to elevations of lung compliance. These adjustments may include increasing the threshold for lung compliance and/or reducing the period of time for which lung compliance must remain below the threshold before pulmonary congestion is identified.

The notification module 40 is configured to notify one or more users (*e.g.,* subject 12, one or more caregivers, *etc*.) of determinations by system 10 related to pulmonary congestion. The notifications generated by notification module 40 include notifications of identifications of pulmonary congestion by congestion monitor module 36. The notifications generated by notification module 40 can be provided in a variety of ways.

For example, in one embodiment, if an identification of pulmonary congestion in subject 12 is made, notification module 40 generates a notification to subject 12 of the identified pulmonary congestion. The notification is provided to subject 12 via user interface 16. For example, the notification may be provided to subject 12 via a user interface provided integrally with pressure support device 18, a user interface provided with processing platform associated with subject 12 (*e.g.,* a desktop computer, a laptop computer, a handheld computer, a mobile communications device, *etc*.), a user interface provided via a website or web portal, and/or another user interface. The notifications generated by notification module 40 for subject 12 may include one or more of an audible notification, a visual notification, a tactile notification, and/or other notifications.

The notification module 40 may be configured to generate notifications to subject 12 of pulmonary congestion that do not merely inform subject 12 of identifications of pulmonary congestion. For example, notifications may provide cues that prompt subject 12 to take action that will reduce the chances of heart failure that often accompanies increases in pulmonary congestion. These actions may include one or more of adjusting medication dosages, scheduling an appointment with a caregiver, interrogating an implantable device for data, triggering an automated detection of one or more physical exam parameters (*e.g.,* auscultation of the lungs or chest, assessing leg/ankle edema, neck vein distension, pulse oximetry, *etc*.), triggering presentation of a set of one or more questions to subject 12 related to severity of symptoms or general health, and/or other actions. The action(s) prompted by notifications generated by notification module 40 for subject 12 may depend on the severity of the pulmonary congestion identified by congestion monitor module 36, the rate at which congestion monitor module 36 has identified pulmonary congestion in subject 12, personal parameters of subject 12 (*e.g.,* height, weight, body mass index, age, medical history, *etc*.), caregiver participation and/or customization, current treatment (*e.g.,* medication and dosage), blood pressure, interrogated device data, and/or other factors.

In addition to providing subject 12 with notifications of identified pulmonary congestion, in one embodiment, notification module 40 is configured to provide subject 12 with historical information. The historical information may include, for example, previous identifications of pulmonary congestion, previous determinations of parameters by parameter module 34, and/or other historical information obtained, determined, and/or managed by the modules of processor 22, and/or trends in such information.

In one embodiment, if an identification of pulmonary congestion in subject 12 is made, notification module 40 generates a notification to a caregiver associated with subject 12. The system 10 may include a module (not shown in FIG. 1) configured to organize notifications provided to a caregiver, such as a doctor. For the doctor, the module may organize the notifications according to subject and/or severity of pulmonary congestion. The notifications may be organized to show trends in pulmonary congestion in individual subjects.

Notifications generated by notification module 40 for a caregiver may be provided to the caregiver via user interface 16. For example, the notifications may be provided to the caregiver via a user interface provided with processing platform associated with the caregiver (*e.g.,* a desktop computer, a laptop computer, a handheld computer, a mobile communications device, *etc.),* a user interface provided via a website or web portal, and/or another user interface. The notifications generated by notification module 40 for the caregiver may include one or more of an audible notification, a visual notification, a tactile notification, and/or other notifications. For instance, in one embodiment, system 10 includes a terminal 56 from which the caregiver accesses the notifications.

Notifications generated by notification module 40 for a caregiver may include, or provide access to, additional information about subject 12 and/or appropriate treatment for subject 12. For example, notifications generated for the caregiver may provide the caregiver with access to previous identifications of pulmonary congestion in subject 12, personal parameters of subject 12, the medical history of subject 12, current treatments of subject 12, and/or other information about subject 12. Access to the additional information may be provided, for instance, in the form of hyperlinks and/or expandable or drop-down menus in a graphical user interface. Such information may be provided to the caregiver to facilitate decisions by the caregiver regarding the treatment of subject 12 in response to the identified pulmonary congestion.

By way of illustration, FIG. 3 provides an example of the manner in which notifications generated by system 10 for the caregiver may be organized. In particular, FIG. 3 illustrates a graphical user interface that conveys the notifications to the caregiver. The notifications are organized by subject, chronology, and/or severity. The graphical user interface illustrated in FIG. 3 may facilitate access by the caregiver about the subjects and/or identifications of pulmonary congestion represented. For example, the caregiver may access additional information about a subject by selecting that subject. The caregiver may access additional information about an identification of pulmonary congestion by selecting a day for the subject.

Returning to FIG. 1, communication module 42 is configured to facilitate communication between subject 12 and a caregiver. The communication may include one or more of email, text messaging, electronic message, text chat, voice chat, instant messaging, network forum, and/or other communication media. The communication may enable the caregiver to instruct subject 12 as to the steps that subject 12 should take to avoid heart failure associated with identified pulmonary congestion.

In one embodiment, communication is generated automatically by communication module 42 upon a determination of pulmonary congestion by congestion monitor module 36. For example, upon a determination of pulmonary congestion by congestion monitor module 36, communication module 42 may automatically generate a communication to subject 12 that identifies a plurality of available appointments with the caregiver. The subject 12 may select a convenient appointment, and this selection may be communicated to the caregiver (and/or his administrative staff) to reserve the appointment for subject 12. As another example, upon a determination of pulmonary congestion by congestion monitor module 36, communication module 42 may automatically generate a communication from the caregiver to subject 12 indicating actions that subject 12 should take to avoid heart failure. The automatically generated communication may be presented to the caregiver (*e.g.,* user interface 16) prior to transmission to subject 12 to enable the caregiver to customize the communication specifically for subject 12.

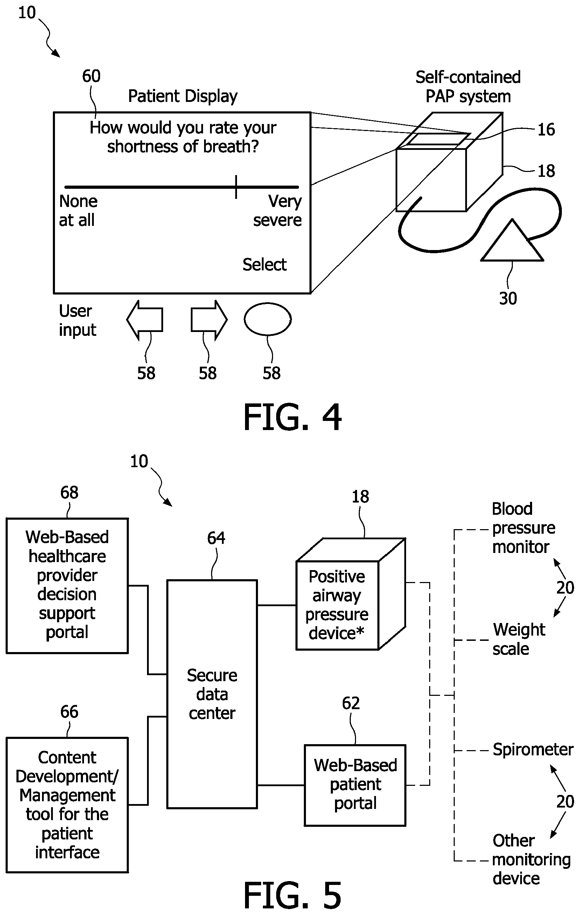

FIG. 4 illustrates an embodiment of system 10, including a specific arrangement of at least some components of system 10, as is discussed further below. This specific embodiment of system 10 is not intended to be limiting, but is only provided for illustrative purposes.

In the embodiment of system 10 illustrated in FIG. 4, system 10 includes pressure support device 18 configured to provide positive airway pressure support to a subject during bedtime through interface appliance 30. The user interface 16 includes an input device 58 (*e.g.,* comprising buttons as shown) and an electronic display 60. Some or all of the functionality attributed to processor 22 (and its modules) is provided by one or more processors provided integrally within pressure support device 18.

Input device 58 is configured to enable a subject to input information to system 10. For example, input device 58 may enable the subject to input some or all of the information discussed above with respect to subject 12 and user interface 16 (shown in FIG. 1 and described above).

The electronic display 60 may be configured to convey information to subject 12. For instance, electronic display 60 may convey some or all of the information to the subject discussed above with respect to subject 12 and user interface 16 (shown in FIG. 1 and described above).

In some implementations, user interface 16, illustrated as being provided integrally in a unitary structure of pressure support device 18, may be provided as a modular unit that is connected with pressure support device 18. The connection between user interface 16 and pressure support device 18 may be selectively removable.

The input device 58 and electronic display 60 also provide an interface for the subject with pressure support device 18. Via this interface, the subject can interact with and/or control the positive airway pressure support therapy provided by pressure support device 18.

FIG. 5 illustrates an embodiment of system 10, including a specific arrangement of at least some components of system 10, as is discussed further below. This specific embodiment of system 10 is not intended to be limiting, but is only provided for illustrative purposes.

In the embodiment illustrated in FIG. 5, system 10 has a distributed architecture. In particular, system 10 includes pressure support device 18, sensors 20, and a web-based portal 62 available to a subject, all of which are accessible by the subject. The system 10 further includes a secure data center 64 and a content development/management tool 66 that are primarily associated (at least on the whole) with a service provider that facilitates the overall operation of system 10 illustrated in FIG. 5. The system 10 further includes a web-based portal 68 available to a caregiver.

As can be appreciated from FIG. 5, the sensors 20 included in system 10 may include sensors external from pressure support device 18. The sensors 20 may further include sensors in communication with gas provided to the subject by pressure support device 18, as was discussed above.

The web-based portal 62 includes an information portal provided to the subject over a network, such as the Internet. The information portal may be established in the form of a web page, or some other type of information portal. The information portal may include a graphical user interface that enables the subject to receive notifications from system 10 (*e.g.,* generated by notification module 40, shown in FIG. 1 and described above), communicate with the caregiver, and/or otherwise interact with system 10. In one embodiment, some or all of the interactions that can be accomplished by the subject may be also be accomplished on a user interface that is provided with pressure support device 18 (not shown in FIG. 5).

The secure data center 64 is a central and secure electronic storage that is configured to store information related to the subject (and other subjects), the caregiver (and other caregivers), system settings, and/or other information related to the operation of system 10. As such, secure data center 64 provides at least some of the functionality attributed to electronic storage 14. The secure data center may be associated with a server that hosts one or both of web-based portal 62 and/or web-based portal 68.

The information stored within secure data center 64 may include information from sensors 20 not associated directly with pressure support device 18. This information may be communicated to secure data center 64 from sensors 20 directly (*e.g.,* via a network connection, via a direct connection, via a wireless connection, via a wired connection, *etc.),* through pressure support device 18, through web-based portal 62 and/or a server associated therewith, or otherwise communicated from sensors 20 to secure data center 64

In one embodiment, secure data center 64 is associated with one or more processors (*e.g.,* one or more servers) that provide at least some of the functionality of the modules of processor 22 (shown in FIG. 1 and described above). These processors manage and organize the storage of information within secure data center 64 and the access of such information through web-based portals 64 and 68.

The content development/management tool 66 provides an interface to system 10 for a third-party service provider that hosts at least some of the components of system 10 (*e.g.,* secure data center 64, web-based portal 62, and/or web-based portal 68). Via content development/management tool 66, the service provider may manipulate the manner in which information within system 10 is processed and/or configure the user interfaces provided by web-based portals 62 and 68.

The web-based portal 68 includes an information portal provided to the caregiver over a network, such as the Internet. The caregiver may access web-based portal 68 via a terminal that is the same as or similar to terminal 56 (shown in FIG. 1 and described above). The information portal may be established in the form of a web page, or some other type of information portal. The information portal may include a graphical user interface that enables the caregiver to receive notifications from system 10 (*e.g.,* generated by notification module 40, shown in FIG. 1 and described above), communicate with the subject, and/or otherwise interact with system 10.

It should be appreciated that although system 10 is described above with respect to FIGS. 1-5 as including pressure support device 18. System 10 may be implemented with out a pressure support device. For example, system 10 may include a spirometer configured to generate output signals conveying information related to the parameters determined by parameter module 34. However, providing pressure support device 18 in system 10 may enhance that passivity of the testing regimen of system 10 with respect to subject 12. Further, at least some of the symptoms of pulmonary congestion may be exacerbated by prolonged periods in the supine position (*e.g.,* at bedtime). Thus, monitoring parameters related to pulmonary congestion via a system that the subject uses at bedtime and/or while sleeping may enhance the accuracy and/or precision of identification of pulmonary congestion in the subject.

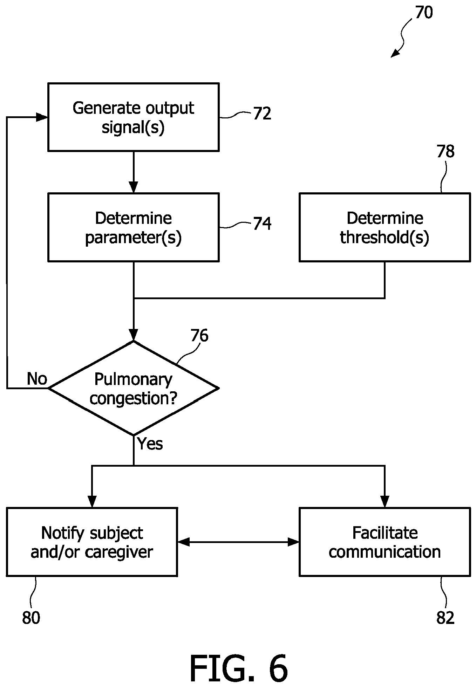

FIG. 6 illustrates a method 70 of monitoring pulmonary congestion in a subject. The operations of method 70 presented below are intended to be illustrative. In some embodiments, method 70 may be accomplished with one or more additional operations not described, and/or without one or more of the operations discussed. Additionally, the order in which the operations of method 70 are illustrated in FIG. 6 and described below is not intended to be limiting. In some embodiments, method 70 may be implemented in a system that is the same as or similar to system 10 (shown in FIGS. 1, 4, and 5). However, in some embodiments, the method 70 may be implemented in other contexts without departing from the scope of this disclosure.

At an operation 72, one or more output signals are generated that convey information related to one or more parameters that are indicative of pulmonary congestion in the subject. The one or more parameters may include at least one parameter of the breathing of the subject. In one embodiment, operation 72 is performed by one or more sensors that are the same as or similar to sensors 20 (shown in FIGS. 1, 4, and 5, and described above).

At an operation 74, the one or more parameters indicated of pulmonary congestion in the subject are determined. In one embodiment, operation 74 is performed by a parameter module that is similar to or the same as parameter module 34 (shown in FIG. 1 and described above).

At an operation 76, one or more thresholds corresponding to the one or more parameters determined at operation 76 are determined. In one embodiment, operation 76 is performed by a threshold module that is the same as or similar to threshold module 38 (shown in FIG. 1 and described above).

At an operation 78, a determination is made as to whether the subject is experiencing pulmonary congestion. The determination made at operation 78 is based on the one or more parameters determined at operation 74. The determination made at operation 78 may be based on a comparison of the one or more parameters with the corresponding thresholds determined at operation 76. In one embodiment, operation 78 is performed by a congestion monitor module that is the same as or similar to congestion monitor module 36 (shown in FIG. 1 and described above).

If pulmonary congestion in the subject is not identified at operation 78, method 70 returns to operation 72. If pulmonary congestion in the subject is identified at operation 78, then method 70 proceeds to operation(s) 80 and/or 82.

At operation 80, one or more notifications of the identified pulmonary congestion are generated. The one or more notifications may include a notification generated for the subject and/or a notification generated for a caregiver. In one embodiment, operation 80 is performed by a notification module that is the same as or similar to notification module 40 (shown in FIG. 1 and described above).

At operation 82, communication between the subject and the caregiver is facilitated. In one embodiment, operation 82 is performed by a communication module that is the same as or similar to communication module 42 (shown in FIG. 1 and described above).

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A system (10) configured to monitor pulmonary congestion in a subject (12), the system comprising:
a pressure generator (18) configured for generating a pressurized flow of breathable gas for delivery to the airway of the subject, said pressure generator (18) comprising a gas support device (28) configured to control one or more of the parameters of the pressurized flow of breathable gas in accordance with a predetermined algorithm;
one or more sensors (20) configured to monitor gas breathed by the subject; a user interface (16) configured to enable a user to interact with the system; and
one or more processors (22) configured to implement a plurality of computer program modules, the computer program modules comprising:
a parameter module (34) configured to receive output signals from the one or more sensors (20) and to determine one or more parameters of the breathing of the subject from the received output signals,
a congestion monitor module (36) configured to identify pulmonary congestion in the subject based on the one or more parameters of the breathing of the subject determined by the parameter module; and
a notification module (40) configured to control the user interface to provide a notification to the user of pulmonary congestion in the subject, if the congestion monitor module identifies pulmonary congestion in the subject;
**characterized in that**
the parameter module (34) is further configured to cause the pressure support device (28) to override the treatment algorithm to facilitate the determination of the one or more parameters of the breathing of the subject according to a predetermined testing regime.

2. The system of claim 1, wherein the user is the subject.

3. The system of claim 1, wherein the user is a caregiver to the subject.

4. The system of claim 3, wherein the user interface includes a graphical user interface that is presented to the user on a terminal located remotely from the one or more sensors.

5. The system of claim 1, wherein the one or more parameters of the breathing of the subject indicate one or both of lung compliance and/or Cheyne-Stokes respiration.

## Patentansprüche

1. System (10), das konfiguriert ist, um Lungenstauung in einer Person (12) zu überwachen, wobei das System Folgendes umfasst:
einen Druckgenerator (18), der konfiguriert ist, um eine unter Druck stehende Strömung von Atemgas zur Abgabe in den Luftweg der Person zu erzeugen, wobei der besagte Druckgenerator (18) eine Gasträgervorrichtung (28) umfasst, die konfiguriert ist, um einen oder mehrere der Parameter der unter Druck stehenden Strömung von Atemgas entsprechend einem vorbestimmten Algorithmus zu steuern;
einen oder mehrere Sensoren (20), die konfiguriert sind, um das Gas zu überwachen, das von der Person eingeatmet wird;
eine Benutzerschnittstelle (16), die konfiguriert ist, um es einem Benutzer zu ermöglichen, mit dem System zu interagieren; und
einen oder mehrere Prozessoren (22), die konfiguriert sind, um eine Vielzahl von Computerprogramm-Modulen zu implementieren, wobei die Computerprogramm-Module Folgendes umfassen:
ein Parametermodul (34), das konfiguriert ist, um Ausgangssignale von dem einen oder mehreren Sensoren (20) zu empfangen, und um einen oder mehrere Parameter der Atmung der Person aus den empfangenen Ausgangssignalen zu bestimmen,
ein Stauungsüberwachungsmodul (36), das konfiguriert ist, um basierend auf dem einen oder mehreren Parametern der Atmung der Person, die durch das Parametermodul bestimmt werden, eine Lungenstauung bei einer Person zu identifizieren; und
ein Benachrichtigungsmodul (40), das konfiguriert ist, um die Benutzerschnittstelle zu steuern, um dem Benutzer eine Benachrichtigung über Lungenstauung in der Person bereitzustellen, falls das Stauungsüberwachungsmodul eine Lungenstauung in der Person feststellt;
**dadurch gekennzeichnet, dass** das Parametermodul (34) darüber hinaus konfiguriert ist, um die Gasträgervorrichtung (28) dazu zu bringen, den Behandlungsalgorithmus zu überschreiben, um die Bestimmung des einen oder mehrerer Parameter der Atmung der Person entsprechend einem vorbestimmten Prüfprogramm zu erleichtern.

2. System nach Anspruch 1, wobei der Benutzer die Person ist.

3. System nach Anspruch 1, wobei der Benutzer eine Pflegeperson der Person ist.

4. System nach Anspruch 3, wobei die Benutzerschnittstelle eine grafische Benutzerschnittstelle enthält, die dem Benutzer auf einem Endgerät präsentiert wird, das von dem einen oder mehreren Sensoren entfernt angeordnet ist.

5. System nach Anspruch 1, wobei der eine oder mehrere Parameter der Atmung der Person entweder auf eine Lungennachgiebigkeit und / oder Cheyne-Stokes-Atmung, oder auf beide hinweist.

## Revendications

1. Système (10) configuré pour surveiller la congestion pulmonaire chez un sujet (12), le système comprenant :
un générateur de pression (18) configuré pour générer un écoulement sous pression de gaz respirable pour administration aux voies respiratoires du sujet, ledit générateur de pression (18) comprenant un dispositif de support de gaz (28) configuré pour commander un ou plusieurs des paramètres de l'écoulement sous pression de gaz respirable conformément à un algorithme prédéterminé ;
un ou plusieurs capteurs (20) configurés pour surveiller le gaz respiré par le suj et ;
une interface utilisateur (16) configurée pour permettre à un utilisateur d'interagir avec le système ; et
un ou plusieurs processeurs (22) configurés pour mettre en oeuvre une pluralité de modules de programmes informatiques, les modules de programmes informatiques comprenant :
un module de paramètres (34) configuré pour recevoir des signaux de sotie provenant du ou des capteurs (20) et pour déterminer un ou plusieurs paramètres de la respiration du sujet à partir des signaux de sortie reçus,
un module de surveillance de congestion (36) configuré pour identifier une congestion pulmonaire chez le sujet sur la base du ou des paramètres de la respiration du sujet déterminés par le module de paramètres ; et
un module de notification (40) configuré pour commander l'interface utilisateur pour fournir une notification l'utilisateur d'une congestion pulmonaire chez le sujet, si le module de surveillance de congestion identifie une congestion pulmonaire chez le sujet ;
**caractérisé en ce que** le module de paramètres (34) est en outre configuré pour amener le dispositif de support de pression (28) à passer outre l'algorithme de traitement afin de faciliter la détermination du ou des paramètres de la respiration du sujet selon un régime de test prédéterminé.

2. Système selon la revendication 1, dans lequel l'utilisateur est le sujet.

3. Système selon la revendication 1, dans lequel l'utilisateur est un soignant du suj et.

4. Système selon la revendication 3, dans lequel l'interface utilisateur inclut une interface utilisateur graphique qui est présentée à l'utilisateur sur un terminal situé à distance du ou des capteurs.

5. Système selon la revendication 1, dans le ou les paramètres de la respiration du sujet indiquent l'une ou les deux parmi une compliance pulmonaire et/ou une respiration de Cheyne-Stokes.
